(19) **Europäisches Patentamt**
**European Patent Office**
**Office européen des brevets**

(11) **EP 1 255 609 B1**

(12) **EUROPÄISCHE PATENTSCHRIFT**

(45) Veröffentlichungstag und Bekanntmachung des Hinweises auf die Patenterteilung:
**15.09.2010 Patentblatt 2010/37**

(21) Anmeldenummer: **01905747.0**

(22) Anmeldetag: **08.02.2001**

(51) Int Cl.:
**B01J 35/02** (2006.01)    **B01J 23/50** (2006.01)
**B01J 23/66** (2006.01)    **C07C 5/02** (2006.01)
**C07C 5/09** (2006.01)

(86) Internationale Anmeldenummer:
**PCT/EP2001/001365**

(87) Internationale Veröffentlichungsnummer:
**WO 2001/058590 (16.08.2001 Gazette 2001/33)**

(54) **KATALYSATOR FÜR DIE HYDRIERUNG VON UNGESÄTTIGTEN KOHLENWASSERSTOFFEN**

CATALYST FOR HYDROGENATING UNSATURATED HYDROCARBONS

CATALYSEUR POUR HYDROGENER DES HYDROCARBURES INSATURES

(84) Benannte Vertragsstaaten:
**AT BE CH CY DE DK ES FI FR GB GR IE IT LI LU MC NL PT SE TR**

(30) Priorität: **10.02.2000 DE 10005775**
**28.09.2000 DE 10048219**

(43) Veröffentlichungstag der Anmeldung:
**13.11.2002 Patentblatt 2002/46**

(73) Patentinhaber: **SÜD-CHEMIE AG**
**80333 München (DE)**

(72) Erfinder:
• **PETROLLI, Mauro**
**81737 München (DE)**
• **GEYER, Ingrid**
**87600 Kaufbeuren (DE)**
• **CASAGRANDE, Francesco**
**Sesia (IT)**

(74) Vertreter: **Dannenberger, Oliver Andre et al**
**Abitz & Partner**
**Patentanwälte**
**Postfach 86 01 09**
**81628 München (DE)**

(56) Entgegenhaltungen:
**EP-A- 0 464 633    EP-A- 0 678 331**
**EP-A- 0 689 872    EP-A- 0 722 776**
**EP-A- 0 780 155**

## Beschreibung

Die Erfindung betrifft einen Katalysator für die Hydrierung von ungesättigten Kohlenwasserstoffen.

Aus der EP-A-0 686 615 ist ein Verfahren zur katalytischen Hydrierung von acetylenischen Verbindungen mit 2 oder 3 Kohlenstoffatomen zu den entsprechenden ethylenischen Verbindungen bekannt, wobei ein Trägerkatalysator in Form von Kugeln oder Extrudaten verwendet wird, der Palladium und Silber enthält. Mindestens 80 % des Palladiums und mindestens 80 % des Silbers liegen nahe an der Peripherie des Katalysators vor. Der Katalysator enthält vorzugsweise Aluminiumoxid als Träger und 0,01 bis 0,5 Gew.-% Palladium und 0,001 bis 0,002 Gew.-% Silber. Aufgrund der Form des Trägers ist die gewichtsbezogene Aktivität und Selektivität relativ gering. Außerdem kann nur mit einer verhältnismäßig geringen Raumgeschwindigkeit gearbeitet werden, und die Druckverluste im Katalysatorbett sind verhältnismäßig hoch.

Aus der EP-A-0 689 872 ist ein Pd und Ag enthaltener Katalysator für die selektive Hydrierung von Acetylen bekannt. Der Katalysator wird dadurch hergestellt, dass ein Trägermaterial (vorzugsweise Aluminiumoxid), in Form von Kugeln oder zylindrischen Tabletten (Pellets) mit einer alkalischen Lösung eines Reduktionsmittels für Pd und Ag behandelt wird.

Aus der EP-A-0 693 315 ist ein Trägerkatalysator für die Diolefin-Hydrierung bekannt, der Palladium, Silber und ein Alkalifluorid enthält. Als Träger werden vorzugsweise sphärische Pellets oder zylindrische Extrudate verwendet.

Aus der EP-A-0. 738 540 ist ein Katalysator für die Hydrierung von $C_2$ bis $C_{10}$-Alkinen (vorzugsweise Acetylen) zu den entsprechenden Alkenen in Gegenwart von Schwefelverbindungen bekannt, der Palladium, Silber, mindestens ein chemisch gebundenes Alkalimetall (vorzugsweise K) chemisch gebundenes Fluor und einen anorganischen Träger (vorzugsweise Aluminiumoxid), in Form von Tabletten enthält.

Die EP-A-0 732 146 beschreibt Katalysatoren, deren Träger Formkörper mit trilobalem Querschnitt und durchgehenden Öffnungen darstellen. Die Katalysatoren enthalten als katalytisch wirksame Komponente Eisenoxid-Molybdänoxid-Verbindungen. Die Katalysatoren werden konkret zur Oxidation von Methanol zu Formaldehyd verwendet, obwohl auch einige andere, nicht durch Beispiele belegte Anwendungen angegeben sind (z.B. die Hydrierung von Acetylen und von Olefinen).

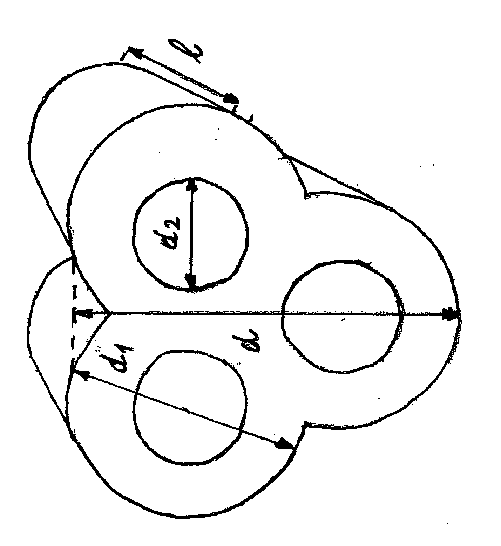

Die EP-A-464 633 beschreibt Katalysatoren, deren Träger Formkörper mit trilobalem Querschnitt und durchgehenden Öffnungen darstellen (vgl. Figur 5). Die Katalysatoren enthalten als katalytisch wirksame Komponenten eine Mischung aus Elementen der Gruppen VIII und 1 B des Periodensystems, insbesondere Palladium und Gold. Sie werden für die Umsetzung von Olefinen mit organischen Carbonsäuren und Sauerstoff zu ungesättigten Estern, insbesondere für die Herstellung von Vinylacetat aus Ethylen und Essigsäure, verwendet.

Die EP-B-591 572 beschreibt ein katalytisches Material in Form von Körnchen mit trilobalem Querschnitt und mindestens drei durchgehenden Porenöffnungen sowie einem bestimmten Verhältnis zwischen der Höhe der Körnchen und dem Abstand zwischen den Achsen der Bohrungen. Dieses Material wird zur oxidativen Dehydrierung von Methanol zur Herstellung von Formaldehyd verwendet.

Ferner werden von der Anmelderin unter den Bezeichnungen G-58 und G-83 Katalysatoren für die selektive Hydrierung von Diolefinen und Alkinen zu Monoolefinen bzw. Alkenen vertrieben, die Palladium und Silber auf einem kugelförmigen Träger oder auf einem Träger in Form von massiven Tabletten oder Extrudaten enthalten. (vgl. Datenblätter "Girdler Catalyst G-58 C, G-58 D, G-58 H, G-58 I and G-83 for Selective Hydrogenation", von Januar 1998).

Katalysatoren auf derartigen Trägern haben im allgemeinen den Nachteil, dass ihre Aktivitäten und Selektivitäten verhältnismäßig niedrig sind und die Hydrierung nur bei verhältnismäßig geringen Raumgeschwindigkeiten durchgeführt werden kann. Außerdem haben diese Katalysatoren einen verhältnismäßig hohen Strömungswiderstand.

Der vorliegenden Erfindung lag die Aufgabe zugrunde, diese Nachteile zu beheben. Es wurde überraschenderweise gefunden, dass diese Nachteile durch Verwendung eines Katalysators behoben werden kann, dessen Träger eine bestimmte Form hat. Gegenstand der Erfindung ist somit ein Katalysator für die Hydrierung von ungesättigten Kohlenwasserstoffen, enthaltend katalytisch wirksame Mengen an Pd und gegebenenfalls Ag auf einem Träger; der Katalysator ist dadurch gekennzeichnet, dass der Träger einen Formkörper mit trilobalem Querschnitt darstellt, wobei die Loben mit durchgehenden Öffnungen versehen sind.

Der erfindungsgemäße Katalysator hat üblicherweise eine geometrische Oberfläche (GO) von etwa 0,2 bis 3 $cm^2$, vorzugsweise von etwa 0.7 bis 1,9 $cm^2$, insbesondere von etwa 0,9 bis 1,5 $cm^2$ pro Formkörper.

Das Verhältnis zwischen der Länge (1) und dem Durchmesser (d) des trilobalen Formkörpers ist vorzugsweise wie folgt:

$$R_i \ = \ l/d \ = \ 2 \ - \ 4$$

Das Verhältnis zwischen geometrischer Oberfläche eines Formkörpers und dem Volumen des festen Anteils eines Formkörpers ($V_f$) beträgt vorzugsweise:

$$R_2 = GO/V_f = 0,5 - 20 \ (mm^{-1}), \ insbesondere \ 1,4 - 4 \ (mm^{-1})$$

Vorzugsweise liegt das Pd in Mengen von etwa 0,01 bis 1,0 Gew.-% und das Ag in Mengen von etwa 0,1 bis 0,5 Gew.-%, bezogen auf das Trägermaterial, vor, und das Gewichtsverhältnis zwischen Pd und Ag beträgt etwa 0,1 bis 5,0.

Die Eindrigtiefe der katalytisch wirksamen Komponente (Pd und gegebenenfalls Ag) in den Träger- Formkörper nach der Reduktion beträgt vorzugsweise etwa 60 bis 300 $\mu$m, bezogen auf 80% der wirksamen Komponente.

Die Kristallitgröße der Pd-Kristallite nach der Reduktion beträgt etwa 2 bis 15 nm, bezogen auf 80% der Pd-Kristallite, und das Verhältnis zwischen der BET-Oberfläche und der Größe der Pd- Kristallite ($d_{Pd}$) ist vorzugsweise wie folgt:

$$R_3 = BET\text{-}O/d_{Pd} = 0,1 - 10$$

Die Größe der Pd-Kristallite wird nach der CO-Adsorptionsmethode in Anlehnung an die Literaturstelle Jounal of Catalysis 25, 148 - 160 (1972) bestimmt.

Als Träger wird vorzugsweise Aluminiumoxid, insbesondere $\theta$-Aluminiumoxid, verwendet. Dieses liegt im allgemeinen nicht in reiner Form vor, sondern kann auch andere Aluminiumoxid-Modifikationen, wie $\alpha$-Aluminiumoxid, enthalten. Es können aber auch Titanoxid, Zirkonoxid, Siliciumdioxid, Zinkoxid, Siliciumcarbid oder Talk verwendet werden.

Die BET-Oberfläche des Trägers beträgt etwa 1 bis 300 m$^2$/g, vorzugsweise etwa 10 bis 300 m$^2$/g, insbesondere etwa 30 bis 80 m$^2$/g. Die BET-Oberfläche wird nach der Einpunkt-Methode durch Adsorption von Stickstoff nach DIN 66132 bestimmt.

Etwa 40% der BET-Oberfläche befinden sich in Poren mit einem Durchmesser von etwa 1570 nm bis 80 nm und etwa 60% befinden sich in Poren mit einem Durchmesser von etwa 80 bis 14 nm. Das Porenvolumen und die Verteilung der spezifischen Oberflächen auf bestimmte Porengrößen wird nach DIN 66133 (Hg-Porosimetrie) bestimmt.

Die erfindungsgemäß als Träger verwendeten trilobalen Formkörper sind in der beigefügten Zeichnung erläutert. Mit (d) ist der Durchmesser, mit (1) die Länge des trilobalen Formkörpers bezeichnet. Mit d1 ist der Durchmesser einer Lobe und mit d2 der Durchmesser der Öffnung in einer Lobe bezeichnet.

Vorzugsweise beträgt der Durchmesser (d) der trilobalen Formkörper etwa 3 bis 10 mm, die Länge (1) etwa 3 bis 15 mm und der Durchmesser einer Öffnung in einer Lobe (d2) etwa 0,5 bis 5 mm.

Die erfindungsgemäßen Katalysatoren enthalten vorzugsweise auch geringe Mengen an Alkali- und/oder Erdalkalimetallen, insbesondere etwa 0,01 bis 0,1 Gew.-% (berechnet als Oxide).

Gegenüber den bekannten Katalysatoren zeichnen sich die erfindungsgemäßen Katalysatoren durch eine hohe Aktivität und Selektivität aus. Außerdem können höhere Raumgeschwindigkeiten von etwa 12000 bis 15000 (Volumteile Reaktanten im gasförmigen Zustand je Volumteil Katalysator und Stunde = GHSV) verwendet werden, verglichen mit einer Raumgeschwindigkeit von nur etwa 3000 bis 8000 bei Verwendung von Kugeln, massiven Tabletten oder Extrudaten. Außerdem zeigen die erfindungsgemäßen Katalysatoren niedrigere Druckverluste (bis 60% gegenüber kugelförmigen oder tablettenförmsgen Trägern (115 bzw. 100%)).

Gegenstand der Erfindung ist ferner ein Verfahren zur Herstellung der vorstehend definierten Katalysatoren, wobei der Träger mit einer Lösung der Salze von Pd und gegebenenfalls Ag ($PdCl_2$, $H_2PdCl_4$ bzw. $AgNO_3$) getränkt wird und die Salze mit Hilfe eines Reduktionsmittels (z.B. Na-Formiat, $NaBH_4$, Hydrazin, Formaldehyd, Ascorbinsäure, Citronensäure, Na-Acetat, Oxalsäure usw.) reduziert werden, und zwar vorzugsweise in einem alkalischen Medium bei Temperaturen zwischen etwa 20 und 100°C, vorzugsweise im Bereich von 40 bis 60°C.

Vorzugsweise führt man die Reduktion in wässrig-alkalischer Lösung durch. Bei dieser Ausführungsform des Verfahrens wird Pd- und ggf. Ag-Oxid an der Oberfläche des Trägers fixiert und dort zu den entsprechenden Metallen reduziert. Auf diese Weise kann die Eindrintiefe der Metalle auf etwa 60 bis 300$\mu$m vermindert werden.

Alternativ kann die Reduktion mit einem Reduktionsmittel in einem nicht-wässrigen Lösungsmittel durchgeführt werden, wenn das Reduktionsmittel durch Wasser zersetzt wird. Dies trifft insbesondere zu für $NaBH_4$, und andere Hydride oder Doppelhydride, wie $LiAlH_4$, $LiBH_4$, $CaH_2$ oder $LaH_3$.

Der imprägnierte Träger wird im allgemeinen gewaschen, getrocknet und calciniert. Falls die Reduktion nicht vollständig ist und Pd und Ag noch teilweise in Form ihrer Oxide vorliegen, werden diese durch Erhitzen in einer wasserstoffhaltigen Atmosphäre zu den entsprechenden Metallen reduziert (Formierung). Die Formierung kann aber auch im Reaktor erfolgen, wobei vor dem Einleiten der zu hydrierenden Verbindungen zunächst nur Wasserstoff eingeleitet wird.

Die Formkörper werden im allgemeinen dadurch hergestellt, dass das Trägermaterial mit Wasser, einem Bindemittel (wie Carboxymethylcellulose) und/oder einem Gleitmittel (z.B. einem Erdalkali- oder Aluminiumstearat) vermischt werden.

Die Herstellung der Formkörper erfolgt in einer Tablettenpresse mit einem Drehteller, an dessen Umfang mehrere Öffnungen mit trilobalem Querschnitt angeordnet sind. In diese Öffnungen (Matrizen) wird die Mischung eingefüllt und von unten von einem Stempel gehalten, durch den während der Drehung des Drehtellers drei Zapfen, die an den Stellen der zu erzeugenden Öffnungen liegen, nach oben geschoben werden. Bei der weiteren Drehung des Drehtellers greift von oben ein Stempel mit trilobalem Querschnitt ein, der mit Öffnungen versehen ist, in die die Zapfen im unteren Stempel beim Herunterdrücken des oberen Stempels eindringen. Die gepreßten Formkörper werden bei der weiteren Drehung des Drehtellers nach dem Zurückziehen des unteren Stempels und dem Weiterschieben des oberen Stempels aus den Matrizen herausgedrückt. Die "grünen" Formkörper werden getrocknet und calciniert. Hierbei entstehen im Formkörper Poren mit der gewünschten Größe.

Die Formkörper werden dann mit einer Lösung der Salze des Palladiums und gegebenenfalls des Silbers getränkt, wobei nach dem Tränken eine alkalische Lösung zugesetzt wird, um das Palladium und das Silber in Form der entsprechenden Oxide auszufällen. Dann wird die Lösung eines Reduktionsmittels zugesetzt, welches die Oxide zu den entsprechenden Metallen reduziert. Es ist aber auch möglich, eine alkalische Reduktionsmittellösung zu verwenden.

Nach der Ausfällung der Oxide bzw. der Metalle der katalytisch wirksamen Komponente(n) werden die Formkörper gewaschen, um lösliche Salze (z.B. NaCl und NaNO$_3$) zu entfernen. Dann werden die Formkörper getrocknet und calciniert, worauf die etwa noch vorhandenen Oxide der katalytisch wirksamen Komponente(n) zu den entsprechenden Metallen reduziert werden. Die Reduktion erfolgt im allgemeinen in einer Wasserstoffatmosphäre bei Temperaturen von etwa 20 bis 450°C. Die Reduktion kann auch im Reaktor erfolgen, indem vor dem Einleiten der zu hydrierenden Verbindungen Wasserstoff oder ein wasserstoffhaltiges Gas eingeleitet wird.

Gegenstand der Erfindung ist schließlich die Verwendung der vorstehend erwähnten Katalysatoren für die Hydrierung von ungesättigten Kohlenwasserstoffen, insbesondere für die selektive Hydrierung von Diolefinen zu Monoolefinen oder von Acetylenen zu Olefinen.

Die Erfindung ist durch die nachstehenden Beispiele erläutert:

Beispiel 1

1000 Gew.-Teile Boehmit werden mit 10 Gew.-Teilen-Wasser und 40 Gew.-Teilen Magnesiumsteart zu einer homogenen, preßbaren Masse vermischt. Diese Masse wird in die Öffnungen des Drehtellers der vorstehend beschriebenen Tablettenpresse gebracht, deren Querschnitte der dargestellten Form entsprechen (d = 6 mm, d1 = 4 mm, D2 = 1,5 mm, 1 = 6 mm). Dann werden wie, oben beschrieben, die Formkörper gepreßt und aus der Tablettenpresse ausgestoßen. Die erhaltenen Formkörper werden zwei Stunden bei 120°C getrocknet und 4,5 Stunden bei 1075°C calciniert, wobei das Boehmit zum größten Teil in θ-Aluminiumoxid (daneben etwas α-Aluminiumoxid) umgewandelt wird. Die geometrische Oberfläche beträgt 1,3 cm$^2$ pro Formkörper. Anschließend wird die BET-Oberfläche nach DIN 66132 zu 30·m$^2$/g bestimmt. Ferner wird nach DIN 66133 das Porenvolumen zu 0,35 ml/g sowie die Porenverteilung bestimmt. 40% der BET-Oberfläche entfallen auf die Poren mit einem Durchmesser von 1750 bis 80 nm, 60% der BET-Oberfäche entfallen auf Poren mit einem Durchmesser von 80 bis 14 nm.

Zu 1000 Gew.-Teilen der Formkörper wird eine H$_2$PdCl$_4$-Lösung (0,345 Gew.-Teile Pd) in 1150 Gew.-Teilen destilliertem Wasser rasch zugegeben und 5 min. langsam gemischt. Man läßt die Mischung etwa 60 min. ruhen und läßt dann die entfärbte Lösung ablaufen.

Das feuchte Produkt wird sofort mit einer etwa 40°C warmen, 5%igen Natriumformiatlösung versetzt und 2,5 Stunden stehen gel assen. Danach wird die Formiatlösung abgelassen, und das Produkt wird mit destilliertem Wasser chloridfrei gewaschen. Anschließend wird das Produkt bei 540°C zwei Stunden calciniert.

1000 Gew.-Teile des calcinierten Produktes werden zu einer Lösung von 3,13 Gew.-Teilen AgNO$_3$ in 1150 Gew.-Teilen destilliertem Wasser gegeben und langsam 5 min. gemischt. Man läßt die Mischung zwei Stunden ruhen und läßt dann die Lösung ablaufen. Das Produkt wird 2,5 Stunden bei 540°C calciniert.

Die so erhaltenen Formkörper werden in einen Röhrenreaktor gefüllt, der zunächst mit Stickstoff gespült wird. Dann wird 8 Stunden Wasserstoff bei einer Temperatur von 400 °C hindurchgeleitet, wodurch das Palladium und das Silber auf den Formkörpern vollständig reduziert werden. Dann wird ein Gemisch aus 1,1 Vol.-% Acetylen und 1,5 Vol.-% Wasserstoff in Ethylen bei einer Temperatur von 50°C, einem Druck von 2,5 MPa und einer Raumgeschwindigkeit von 14000 Liter Gemisch je kg Katalysator und Stunde durch den Reaktor geleitet. Das Acetylen wird zu 84% mit einer Selektivität von 93% in Ethylen umgewandelt.

Vergleichsbeisipiel 1

Ein kugelförmiger Katalysatorträger aus θ-Al$_2$O$_3$ mit einem Durchmesser von 2 bis 4 mm, einer geometrischen Oberfläche von 0,3 cm$^2$, einer BET-Oberfläche von 30 m$^2$/g, einem Porenvolumen von 0,39 ml/g und einer Porenvolumenverteilung von 40% zwischen 1750 und 80 nm und 60% zwischen 80 und 14 nm wird mit der H$_2$PdCl$_4$/AgNO$_3$-Lösung von Beispiel 1 imprägniert und in derselben Weise wie in Beispiel 1 angegeben, reduziert, getrocknet, calciniert und formiert. Der Katalysator wird wie nach Beispiel 1 zur selektiven Hydrierung von Acetylen in Ethylen verwendet, wobei die gleichen Verfahrensbedingungen angewendet werden. Bei einer Raumgeschwindigkeit von 8000 (Liter Reaktanten pro Liter Katalysator und Stunde) wurde das Acetylen zu 57% mit einer Selektivität von 65 % in Ethylen umgewandelt.

Vergleichsbeispiel 2

Die Arbeitsweise von Vergleichsbeispiel 1 wurde mit der Abweichung wiederholt, dass ein Katalysator in Form von 4 x 4 mm-. Tabletten verwendet wurde, wobei alle anderen Bedingungen unverändert blieben. Das Acetylen wurde zu 48% mit einer Selektivität von 76% in Ethylen umgewandelt.
Die Katalysatoren nach Beispiel 1 und nach den Vergleichsbeispielen 1 und 2 wurden in einem getrennten Druckverlustrohr vermessen (mit Stickstoff als Meßgas), wobei folgende Druckverlustrelation ermittelt wurde: Beispiel 1: 60%; Vergleichsbeispiel 1: 115%; Vergleichsbeispiel 2: 100%.

Beispiel 2

Die Arbeitsweise von Beispiel 1 wurde mit der Abweichung wiederholt, dass der Träger 4,5 Stunden bei 1020°C calciniert und der so behandelte Träger mit 0,3 Gew.-% Palladium belegt wurde. Der Katalystator hatte eine BET-Oberfläche vom 70 ± 5 cm$^2$/g ein Porenvolumen von 0,4 ml/g mit 4,71% der Oberfläche in Poren mit einem Durchmesser von 1750 bis 80 nm und 76% in Poren mit einem Durchmesser von 80 bis 14 nm. Der Rest der Oberfläche entfiel auf Poren mit einem Durchmesser von < 14 nm.
Die Katalysator-Formkörper wurden wie nach Beispiel 1 in einem Röhrenreaktor 8 Stunden bei 400°C formiert und mit einem Gemisch aus Wasserstoff und dienhaltigem Pyrolysebenzin (2 Mol H$_2$,1 Mol Dien) zuerst bei 30°C und dann bei 60°C, einem Druck von 3,0 MPa und einer Raumgeschwindigkeit von 8 Volumteilen flüssigem Pyrolysebenzin je Volumteil Katalysator und Stunde (LHSV) beaufschlagt. Die Zusammensetzung des Pyrolysebenzins vor der selektiven Hydrierung ist in Tabelle I angegeben.

<div align="center">

Tabelle I

| | mol% |
|---|---|
| Benzol | 38,6 |
| Toluol | 22,6 |
| o-Xylol | 1,7 |
| p-Xylol | 4,1 |
| m-Xylol | 2,0 |
| Ethylbenzol | 2,0 |
| Styrol | 6,0 |
| Cyclohexan | 0,7 |
| Methylcyclohexan | 1,5 |
| Hexadien | 1,2 |
| Cyclohexadien | 0,3 |
| Hexen | 10,2 |
| Heptan | 1,4 |
| Heptan | 8,9 |

</div>

Die Styrol- und Dien-Umsätze sowie die Dien-Selektivität nach der selektiven Hydrierung sind in Tabelle II angegeben.

TABELLE II

| Stunden h | Temp. °C | Styrol-Umsatz | Dien-Umsatz | Dien-Selektivität |
|---|---|---|---|---|
| 24 | 30 | 98,6 | 99,2 | 81,3 |
| 48 | 30 | 98,6 | 98,9 | 81,0 |
| 72 | 30 | 98,9 | 99,5 | 79,8 |
| 96 | 60 | 100 | 100 | 70,7 |
| 144 | 60 | 100 | 99,8 | 69,8 |
| 170 | 60 | 100 | 99,8 | 69,2 |

Vergleichsbeispiel 2

Die Arbeitsweise von Beispiel 2 wurde mit der Abweichung wiederholt, dass ein Katalysator im Form von Kugeln mit einem Durchmesser von 2 bis 4 mm, mit einer Palladium-Belegung von 0,3% ,einer geometrischen Oberfläche vom 0,3 $cm^2$, einer BET-Oberfläche von 70 $\pm$ 5 $cm^2$/g, einem Porenvolumen von 0,5 ml/g und einer Porenverteilung wie bei dem Katalysator von Beispiel 2, mit Pyrolysebenzin mit der in Tabelle I angegebenen Zusammensetzung bei einer Temperatur von anfangs 30°C und später von 60°C, einem Druck von 3,0 MPa und einer LSHV von 4 beaufschlagt wurde. Die Styrol- und Dien-Umsätze sowie die Dien-Selektivität nach der selektiven Hydrierung sind in Tabelle III angegeben.

Tabelle III

| Stunden h | Temp. °C | Styrol-Umsatz | Dien-Umsatz | Dien-Selektivität |
|---|---|---|---|---|
| 24 | 30 | 83,7 | 81,7 | 60,9 |
| 48 | 30 | 84,5 | 81,7 | 59,6 |
| 72 | 30 | 81,7 | 81,9 | 57,9 |
| 96 | 60 | 95,7 | 91,7 | 50,3 |
| 144 | 60 | 95,6 | 91,5 | 45,5 |
| 170 | 60 | 95,7 | 91,8 | 40,8 |

Beispiel 3

In Anlehnung an die Arbeitsweise von Beispiel 1 der EP-0 314 024-A1 wurde ein kommerzielles Titandioxid (P-25 Degussa) in einem Intensivmischer unter Zusatz von etwa 55 Gew.-% Wasser und 14 Gew.-% Isopropyltitanat 45 min. homogenisiert. Nach mehrstündigem Trocknen bei 110°C wird die $TiO_2$-Masse zerkleinert, mit Aluminiumstearat als Tablettierungsmittel vermischt und wie nach Beispiel 1 zu Formkörpern mit trilobalem Querschnitt gepreßt (Abmessungen wie in Beispiel 1).

Die $TiO_2$-Formkörper wurden 3 Stunden bei 550°C in oxidierender Atmosphäre calciniert.

Die geometrische Oberfläche des Trägers betrug 1,3 $cm^2$ pro Formkörper, die BET-Oberfläche 36 $m^2$/g, das Porenvolumen 0,39 ml/g. 3,7 % der BET-Oberfläche entfielen auf Poren mit einem Durchmesser von 1750 bis 80 nm, 95,8 % auf Poren mit einem Durchmesser von 80 bis 14 nm.

Der $TiO_2$-Träger wurde mit einer 8%-igen wäßrigen Natriumformiatlösung sprühimprägniert (auf 100 g Träger 30 ml Formiatlösung). Dann wurde der so vorbehandelte Träger mit dem gleichem Volumen einer 2,5%-igen wäßrigen $PdCl_2$-Lösung sprühimprägniert. Zur vollständigen Reduktion des Edelmetalls wurden die Träger-Formkörper mit Formiatlösung überschichtet, abgesaugt und chloridfrei gewaschen. Nach dem Trocknen bei 100°C wurde 6 Stunden bis zu einer Endtemperatur von 400 °C calciniert. Anschließend wurde die Promotierung mit Silber vorgenommen. Hierzu wurden die palladiumhaltigen $TiO_2$-Formkörper bei Raumtemperatur mit Silbernitratlösung imprägniert, bei 110°C getrocknet und nochmals 6 Stunden bis zu einer Temperatur von 360°C calciniert. Der Palladiumgehalt betrug 0,21 Gew.-%, der Silbergehalt 0,12 Gew.-%.

Der Katalysator wurde bei einem Druck von 0,15 MPa, einer Temperatur von 120°C und einer LHSV von 15 $h^{-1}$ mit einem Molverhältnis $H_2$/Dien von 2 : 1 zur selektiven Hydrierung eines flüssigen dienhaltigen Gemischs mit der Zusammensetzung

- 85,7 mol-% Paraffinen
- 11,1 mol-% Mono-Olefinen

- 0,85 mol-% Dienen
- 2,40 mol-% Aromaten

verwendet.

Der Dien-Umsatz betrug 80% bei einer Selektivität von 85%.

Vergleichsbeispiel 3

Die Arbeitsweise von Beispiel 3 wurde mit der Abweichung wiederholt, dass statt der trilobalen Formkörper massive Tabletten mit den Abmessungen 4,5 x 4,5 mm verwendet werden, die in der gleichen Weise wie nach Beispiel 3 mit den katalytisch wirksamen Metallen belegt wurden. Der Träger hatte eine BET-Oberfläche von 33 m$^2$/g und ein Porenvolumen von 0,2 ml/g. 51% der BET-Oberfläche entfielen auf Poren mit einem Durchmesser von 1750 bis 80 nm, 87,3% auf Poren mit einem Durchmesser von 80 bis 14 nm. Der Katalysator enthielt 0,213 Gew.-% Pd und 0,27 Gew.-% Ag.

Der Katalysator wurde zur selektiven Hydrierung des in Beispiel 3 verwendeten dienhaltigen Gemischs verwendet (LHSV = 10 h$^{-1}$, T = 120°C, Druck = 0,15 MPa, Molverhältnis H$_2$:Dien = 4 : 1).

Der Dien-Umsatz betrug 70% bei einer Selektivität von 60%

**Patentansprüche**

1. Verwendung eines Katalysators, enthaltend katalytisch wirksame Mengen an Pd und gegebenenfalls Ag auf einem Träger, wobei der Träger einen Formkörper mit trilobalem Querschnitt darstellt, die Loben mit durchgehenden Öffnungen versehen sind und die geometrische Oberfläche (GO) 0,2 bis 3 cm$^2$ pro Formkörper, der Durchmesser (d) der trilobalen Formkörper 3 bis 10 mm, die Länge (1) 3 bis 15 mm und der Durchmesser einer Öffnung in einer Lobe (d2) 0,5 bis 5 mm beträgt für die Hydrierung von ungesättigten Kohlenwasserstoffen, insbesondere für die selektive Hydrierung von Diolefinen zu Monoolefinen oder von Acetylenen zu Olefinen.

2. Verwendung nach Anspruch 1, **dadurch gekennzeichnet, dass** das Verhältnis zwischen der Länge (1), und dem Durchmesser (d) des trilobalen Formkörpers wie folgt ist:

$$R_1 = l/d = 2 - 4.$$

3. Verwendung nach Anspruch 1 oder 2, **dadurch gekennzeichnet, dass** die geometrische Oberfläche (GO) 0,7 bis 1,2 cm$^2$, insbesondere 0,9 bis 1,5 cm$^2$ pro Formkörper beträgt.

4. Verwendung nach einem der Ansprüche 1 bis 3, **dadurch gekennzeichnet, dass** das Verhältnis zwischen der geometrischen Oberfläche eines Formkörpers und dem Volumen des festen Anteils eines Formkörpers (Vf)

$$R_2 = GO/V_f = 0,5 - 20 \ (mm^{-1}), \ vorzugsweise \ 1,4 - 4 \ (mm^{-1})$$

beträgt.

5. Verwendung nach einem der Ansprüche 1 bis 4, **dadurch gekennzeichnet, dass** das Pd in Mengen von 0,01 bis 1,0 Gew. und das Ag in Mengen von 0,1 bis 0,5 Gew.-%, bezogen auf das Trägermaterial, vorliegt, und dass das Gewichtsverhältnis zwischen Pd und Ag 0,1 bis 5,0 beträgt.

6. Verwendung nach einem der Ansprüche 1 bis 5, **dadurch gekennzeichnet, dass** die Kristallitgröße der Pd-Kristallite nach der Reduktion 2 - 15 nm, (Bezogen auf 80% der Pd-Kristallite), beträgt.

7. Verwendung nach einem der Ansprüche 1 bis 6, **dadurch gekennzeichnet, dass** das Verhältnis zwischen der BET-Oberfläche und der Größe der Pd-Kristallite (d$_{Pd}$) wie folgt ist

$$R_3 = BET\text{-}O/d_{Pd} = 0,1 - 10.$$

**8.** Verwendung nach einem der Ansprüche 1 bis 7, **dadurch gekennzeichnet, dass** der Träger Aluminiumoxid, insbesondere
θ - Aluminiumoxid, darstellt.

**9.** Verwendung nach einem der Ansprüche 1 bis 8, **dadurch gekennzeichnet, dass** die BET-Oberfläche des Trägers 1 bis 300 m$^2$/g, vorzugsweise 10 bis 300 m$^2$/g, insbesondere 30 bis 80 m$^2$/g, beträgt.

**10.** Verwendung nach einem der Ansprüche 1 bis 9, **dadurch gekennzeichnet, dass** 40 % der BET-Oberfläche sich in Poren mit einem Durchmesser von 1.750 bis 80 nm, und 60 % sich in Poren mit einem Durchmesser von 80 bis 14 nm befindet.

**11.** Verwendung nach einem der Ansprüche 1 bis 10, **dadurch gekennzeichnet, dass** der Katalysator auch geringe Mengen an Alkali- und/oder Erdalkalimetallen, vorzugsweise 0,01 bis 0,1 Gew.-% (berechnet als Oxide) enthält.

**12.** Verwendung nach einem der Ansprüche 1 bis 11, **dadurch gekennzeichnet, dass** der Katalysator nach einem Verfahren hergestellt worden ist, bei dem der Träger mit einer Lösung der Salze von Pd und gegebenenfalls Ag getränkt und diese Salze mit Hilfe eines Reduktionsmittels reduziert worden sind, worauf der so imprägnierte Träger gewaschen, getrocknet und calciniert worden ist und, wenn die Reduktion nicht vollständig ist, die noch vorhandenen Oxide des Pd und Ag durch Erhitzen in einer wasserstoffhaltigen Atmosphäre zu den entsprechenden Metallen reduziert worden sind.

**13.** Verwendung nach Anspruch 12, **dadurch gekennzeichnet, dass** die Reduktion in einem alkalischen Medium bei Temperaturen zwischen 20 und 100°C, vorzugsweise im Bereich von 40 bis 80°C durchführt worden ist.

**14.** Verwendung nach Anspruch 12 oder 13, **dadurch gekennzeichnet, dass** die Reduktion in wässrig-alkalischer Lösung durchgeführt worden ist.

**Claims**

**1.** Use of a catalyst, containing catalytically effective quantities of Pd and optionally Ag on a support, wherein the support represents a shaped body with a trilobal cross section, the lobes are provided with continuous openings and the geometric surface area (GS) is 0.2 to 3 cm$^2$ per shaped body, the diameter (d) of the trilobal shaped bodies is 3 to 10 mm, the length (1) is 3 to 15 mm and the diameter of one opening in one lobe (d2) is 0.5 to 5 mm, for the hydrogenation of unsaturated hydrocarbons, in particular for the selective hydrogenation of diolefins to monoolefins or of acetylenes to olefins.

**2.** Use according to claim 1, **characterized in that** the relationship between the length (1) and the diameter (d) of the trilobal shaped body is as follows:

$$R_1 = l/d = 2 - 4.$$

**3.** Use according to claim 1 or 2, **characterized in that** the geometric surface area (GS) is 0.7 to 1.2 cm$^2$, in particular 0.9 to 1.5 cm$^2$ per shaped body.

**4.** Use according to one of claims 1 to 3, **characterized in that** the relationship between the geometric surface area of a shaped body and the volume of the solid portion of a shaped body (Vs) is

$$R_2 = GS/V_s = 0.5 - 20 \ (mm^{-1}), \text{ preferably } 1.4 - 4 \ (mm^{-1}).$$

**5.** Use according to one of claims 1 to 4, **characterized in that** the Pd is present in quantities of 0.01 to 1.0 wt.-% and the Ag in quantities of 0.1 to 0.5 wt.-%, relative to the support material, and that the weight ratio between Pd and Ag is 0.1 to 5.0.

6. Use according to one of claims 1 to 5, **characterized in that** the crystallite size of the Pd crystallites after the reduction is 2-15 nm (based on 80% of the Pd crystallites).

7. Use according to one of claims 1 to 6, **characterized in that** the relationship between the BET surface area and the size of the Pd crystallites ($d_{Pd}$) is as follows

$$R_3 = BET\text{-}S/d_{Pd} = 0.1 - 10.$$

8. Use according to one of claims 1 to 7, **characterized in that** the support represents aluminium oxide, in particular θ-aluminium oxide.

9. Use according to one of claims 1 to 8, **characterized in that** the BET surface area of the support is 1 to 300 $m^2/g$, preferably 10 to 300 $m^2/g$, in particular 30 to 80 $m^2/g$.

10. Use according to one of claims 1 to 9, **characterized in that** 40% of the BET surface area is located in pores with a diameter of 1750 to 80 nm, and 60% in pores with a diameter of 80 to 14 nm.

11. Use according to one of claims 1 to 10, **characterized in that** the catalyst also contains small quantities of alkali and/or alkaline earth metals, preferably 0.01 to 0.1 wt.-% (calculated as oxides).

12. Use according to one of claims 1 to 11, **characterized in that** the catalyst was prepared according to a process in which the support was soaked with a solution of the salts of Pd and optionally of Ag and these salts were reduced using a reducing agent, whereupon the thus-impregnated support was washed, dried and calcined and, if the reduction is not complete, the oxides of Pd and Ag still present were reduced to the corresponding metals by heating in a hydrogen-containing atmosphere.

13. Use according to claim 12, **characterized in that** the reduction was carried out in an alkaline medium at temperatures between 20 and 100°C, preferably in the range from 40 to 80°C.

14. Use according to claim 12 or 13, **characterized in that** the reduction was carried out in aqueous-alkaline solution.

**Revendications**

1. Utilisation d'un catalyseur, contenant des quantités actives du point de vue catalytique de Pd et, le cas échéant, d'Ag sur un support, où le support représente un corps de forme ayant une section transversale trilobée, les lobes sont pourvues d'ouvertures de passage et la surface géométrique (SG) est de 0,2 à 3 $cm^2$ par corps de forme, le diamètre (d) du corps de forme trilobé est de 3 à 10 mm, la longueur (1) est de 3 à 15 mm et le diamètre d'une ouverture dans un lobe (d2) est de 0,5 à 5 mm, en vue de l'hydrogénation d'hydrocarbures insaturés, en particulier en vue de l'hydrogénation sélective de dioléfines en mono-oléfines ou d'acétylènes en oléfines.

2. Utilisation selon la revendication 1, **caractérisée en ce que** le rapport entre la longueur (1) et le diamètre (d) du corps de forme trilobé est comme suit :

$$R_1 = 1/d = 2 - 4.$$

3. Utilisation selon la revendication 1 ou 2, **caractérisée en ce que** la surface géométrique (SG) est de 0,7 à 1,2 $cm^2$, en particulier de 0,9 à 1,5 $cm^2$ par corps de forme.

4. Utilisation selon l'une quelconque des revendications 1 à 3, **caractérisée en ce que** le rapport entre la surface géométrique d'un corps de forme et le volume de la partie solide d'un corps de forme (Vs) est

$$R_2 = SG/V_s = 0,5 - 20 \ (mm^{-1}), \ \text{de préférence} \ 1,4 - 4 \ (mm^{-1}).$$

5.  Utilisation selon l'une quelconque des revendications 1 à 4, **caractérisée en ce que** le Pd est présent dans des quantités de 0,01 à 1,0 % en poids et l'Ag l'est dans des quantités de 0,1 à 0,5 % en poids, par rapport au matériau du support et **en ce que** le rapport pondéral entre le Pd et l'Ag est de 0,1 à 5,0.

6.  Utilisation selon l'une quelconque des revendications 1 à 5, **caractérisée en ce que** la taille de cristallite des cristallites de Pd après la réduction est de 2 - 15 nm (par rapport à 80% des cristallites de Pd).

7.  Utilisation selon l'une quelconque des revendications 1 à 6, **caractérisée en ce que** le rapport entre la surface spécifique BET et la taille des cristallites de Pd ($d_{Pd}$) est comme suit :

$$R_3 = S\text{-}BET/d_{Pd} = 0,1 - 10.$$

8.  Utilisation selon l'une quelconque des revendications 1 à 7, **caractérisée en ce que** le support est constitué d'oxyde d'aluminium, en particulier d'oxyde d'aluminium thêta.

9.  Utilisation selon l'une quelconque des revendications 1 à 8, **caractérisée en ce que** la surface spécifique BET du support est de 1 à 300 m²/g, de préférence de 10 à 300 m²/g, en particulier de 30 à 80 m²/g.

10. Utilisation selon l'une quelconque des revendications 1 à 9, **caractérisée en ce que** 40% de la surface spécifique BET se trouvent dans des pores ayant un diamètre de 1.750 à 80 nm, et 60% de cette dernière se trouvent dans des pores ayant un diamètre de 80 à 14 nm.

11. Utilisation selon l'une quelconque des revendications 1 à 10, **caractérisée en ce que** le catalyseur contient aussi de faibles quantités de métaux alcalins et/ou alcalinoterreux, de préférence de 0,01 à 0,1 % en poids (calculés en tant qu'oxydes).

12. Utilisation selon l'une quelconque des revendications 1 à 11, **caractérisée en ce que** le catalyseur est fabriqué conformément à un procédé lors duquel le support a été imbibé d'une solution de sels de Pd et, le cas échéant, d'Ag et ces sels ont été réduits à l'aide d'un agent de réduction, à la suite de quoi le support ainsi imprégné a été lavé, séché et calciné et, si la réduction n'est pas complète, les oxydes de Pd et d'Ag encore présents ont été réduits aux métaux correspondants par chauffage dans une atmosphère contenant de l'hydrogène.

13. Utilisation selon la revendication 12, **caractérisée en ce que** la réduction a été effectuée dans un milieu alcalin à des températures comprises entre 20 et 100°C, de préférence dans le domaine allant de 40 à 80°C.

14. Utilisation selon la revendication 12 ou 13, **caractérisée en ce que** la réduction a été effectuée dans une solution alcaline aqueuse.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0686615 A
- EP 0689872 A
- EP 0693315 A
- EP 0738540 A

- EP 0732146 A
- EP 464633 A
- EP 591572 B
- EP 0314024 A1

**In der Beschreibung aufgeführte Nicht-Patentliteratur**

- Die Größe der Pd-Kristallite wird nach der CO-Adsorptionsmethode in Anlehnung an die Literaturstelle. *Jounal of Catalysis,* 1972, vol. 25, 148-160